(19) Europäisches Patentamt
European Patent Office
Office européen des brevets

(11) Numéro de publication : **0 341 164 B1**

# (12) FASCICULE DE BREVET EUROPEEN

(45) Date de publication du fascicule du brevet : **26.08.92 Bulletin 92/35**

(51) Int. Cl.$^5$ : **C07C 39/08,** C07C 37/60, C01B 21/22

(21) Numéro de dépôt : **89420155.7**

(22) Date de dépôt : **27.04.89**

## (54) Procédé de préparation de dihydroxybenzènes.

(30) Priorité : **02.05.88 FR 8805849**

(43) Date de publication de la demande : **08.11.89 Bulletin 89/45**

(45) Mention de la délivrance du brevet : **26.08.92 Bulletin 92/35**

(84) Etats contractants désignés : **AT BE CH DE ES FR GB GR IT LI LU NL SE**

(56) Documents cités :
**US-A- 1 547 725**
**US-A- 4 578 521**
**PATENT ABSTRACTS OF JAPAN tome 10, no. 31(C-327)(2088), 6 fevrier 1986; & JP - A - 60 184 036**

(56) Documents cités :
**THE JOURNAL OF PHYSICAL CHEMISTRY tome 87, no.6, 17 mars 1983, pages 903-905; M.IWAMOTO et al.: "Catalytic Oxidation by OxideRadical Ions. 1. One-Step Hydroxylation of Benzene to Phenol over Group 5 and 6 Oxides Supported on Silica Gel"**

(73) Titulaire : **RHONE-POULENC CHIMIE**
**25, quai Paul Doumer**
**F-92408 Courbevoie Cédex (FR)**

(72) Inventeur : **Gubelmann, Michel**
**39, Boulevard des Belges**
**F-69006 Lyon (FR)**
Inventeur : **Tirel, Philippe-Jean**
**40, Boulevard Kennedy**
**F-69600 Oullins (FR)**

(74) Mandataire : **Dutruc-Rosset, Marie-Claude et al**
**RHONE-POULENC CHIMIE Direction des Brevets 25, Quai Paul Doumer**
**F-92408 Courbevoie Cédex (FR)**

EP 0 341 164 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation de dihydroxybenzènes. Elle concerne plus particulièrement un procédé d'hydroxylation de phénols.

L'hydroxylation du phénol ou de phénols substitués, par le péroxyde d'hydrogène, pour préparer des diphénols est une réaction connue.

Le brevet français FR 2 071 464 a décrit un procédé dans lequel la réaction est catalysée par un acide fort, tel que par exemple l'acide perchlorique ou l'acide sulfurique.

Le brevet allemand n° 2 410 742 a décrit un procédé semblable au précédent, dans lequel le peroxyde d'hydrogène est mis en oeuvre sous forme de solution organique pratiquement anhydre.

Ces deux procédés sont très intéressants et le premier procédé est mis en oeuvre industriellement.

Cependant depuis quelques années, on cherche à catalyser la réaction d'hydroxylation à l'aide de solides non dissous dans le milieu, afin de simplifier leur séparation du milieu réactionnel et leur éventuel recyclage et éviter les sous-produits salins, qui se forment le plus souvent lors de l'élimination des catalyseurs acides dissous.

On a décrit dans le brevet américain 4 578 521 un procédé de préparation d'hydroquinone qui consiste à faire réagir le phénol avec le peroxyde d'hydrogène, en présence d'un catalyseur qui est une zéolithe, notamment la zéolithe ZSM-5.

Par ailleurs, le brevet français FR 2 489 816 préconise l'emploi de silicalite de titane comme catalyseur hétérogène de l'hydroxylation de composés aromatiques par le peroxyde d'hydrogène.

En fait, cette catalyse présente de grandes difficultés de reproductibilité. En outre la fine taille des particules de catalyseur utilisées rend leur séparation du milieu réactionnel très difficile et leur recyclage problématique, alors que dans un procédé industriel, il est indispensable de pouvoir recycler un catalyseur coûteux.

Pour remédier à ce problème de la séparation du catalyseur, il a été proposé, dans la demande de brevet européen publiée sous le n° 200 260, d'utiliser des agglomérats de ces fines particules de silicalite de titane.

La difficulté de contrôler la sécurité des ateliers lors de l'utilisation de péroxyde d'hydrogène et la médiocrité des rendements obtenus dans l'art antérieur fait que depuis de nombreuses années, le chimiste cherche à introduire directement un groupe hydroxyle sur le noyau phénolique en l'abscence de tout dérivé péroxydique. Cette chimie a longtemps échoué.

Il est également connu selon le brevet américain 1 547 725, de préparer du phénol à partir du benzène en mélangeant ce dernier avec des oxydes d'azote puis en chauffant à une température voisine de 700°C.

La seule publication récente décrivant l'introduction directe d'un groupe hydroxyle sur un noyau benzénique consiste en un article de IWAMOTO paru dans le Journal of Physical Chemistry, 87, 6, pages 903-905 (1983).

Cette réaction d'hydroxylation du benzène est réalisée par le protoxyde d'azote ($N_2O$) en présence d'un catalyseur à base d'un oxyde d'un métal des groupes V ou VI de la classification périodique.

L'oxyde de vanadium est l'oxyde préféré parmi les oxydes des métaux des groupes V et VI de la classification. Il est préférable d'utiliser cet oxyde disposé sur un support à base de silice en quantité pondérale comprise entre 1 et 10 % par rapport au support. Le support est constitué de préférence de silice, l'alumine provoquant majoritairement la formation d'un mélange d'oxydes de carbone.

Le procédé de IWAMOTO était intéressant mais l'utilisation de ces catalyseurs le rendait peu attrayant pour l'industrie.

L'industrie chimique est donc toujours à la recherche d'un procédé d'hydroxylation directe du noyau benzénique sur un substrat aussi simple et facilement disponible qu'est le phénol.

La présente invention a permis d'atteindre cet objectif.

Elle a pour objet un procédé de préparation de dihydroxy benzènes caractérisé en ce que l'on met en présence des phénols et du protoxyde d'azote sur une zéolithe acidifiée.

La zéolithe est choisie notamment parmi les formes commerciales telles que :

. la zéolithe ZSM-5 de MOBIL OIL dont la préparation est décrite dans le brevet US 3702886 ,
. la zéolithe US-Y vendue par TOYO-SODA
. la zéolithe HY vendue par UNION CARBIDE CHEMICALS sous la référence LZY 82.
. la zéolithe H-Mordénite vendue par LA GRANDE PAROISSE.

On préfére utiliser la zéolithe commerciale ZSM-5.

La zéolithe a de préférence un rapport $SiO_2/Al_2O_3$ supérieur à 90 et encore plus préférentiellement compris entre 90 et 500.

La zéolithe commerciale est, de préférence dans le procédé de l'invention, acidifiée par addition d'un acide inorganique choisi parmi l'acide chlorhydrique, l'acide sulfurique, l'acide nitrique, l'acide perchlorique et l'acide phosphorique ou d'un acide organique choisi parmi les acides halosulfoniques, halométhanesulfoniques, halocarboxyliques et de préférence l'acide trifluorométhanesulfonique.

Selon un mode de mise en oeuvre préférentiel, la zéolithe est acidifiée par passage d'un volume d'acide présentant une normalité comprise entre 0,1 N et 2 N par gramme de zéolithe compris entre 10 ml/g et 100 ml/g. Ce passage peut être réalisé en une seule étape ou de préférence en plusieurs étapes successives.

Le protoxyde d'azote est utilisé pur ou en mélange avec un gaz inerte ne contenant pas d'oxygène tel que l'azote.

Le phénol est de préférence introduit en mélange avec le protoxyde d'azote selon un rapport molaire du protoxyde d'azote par rapport au phénol compris entre 1 et 10.

Selon un procédé de mise en oeuvre préférentiel, on vaporise le phénol, on le mélange avec le protoxyde d'azote selon les proportions préalablement définies et on le fait circuler sur la zéolithe. La réaction se déroule de préférence à une température comprise entre 300 et 500°C.

Les gaz issus de la réaction contenant un mélange de dihydroxybenzènes : hydroquinone, pyrochatecol, resorcinol sont condensés et séparés par toute technique connue de l'homme de l'art.

Les exemples suivants donnés uniquement à titre indicatif et nullement limitatif permettent d'illustrer l'invention.

Dans les exemples, les abréviations suivantes signifient :

$$TT = \text{taux de transformation} = \frac{\text{produit transformé}}{\text{produit introduit}} \text{ en moles}$$

$$RT = \text{rendement sur produit transformé} = \frac{\text{produit désiré}}{\text{produit transformé}} \text{ en moles}$$

EXEMPLE

Préparation du catalyseur

On met en contact 10 g de zéolithe commerciale NaZSM5 avec 100 ml d'une solution d'HCl 1N à 60°C pendant 4 heures sous agitation. On laisse refroidir et on lave à l'eau permutée. On filtre le solide et on le sèche à l'étuve à 100°C.

Le lavage ci-dessus décrit est répété 4 fois. Le produit séché obtenu après le 4ème lavage est broyé.

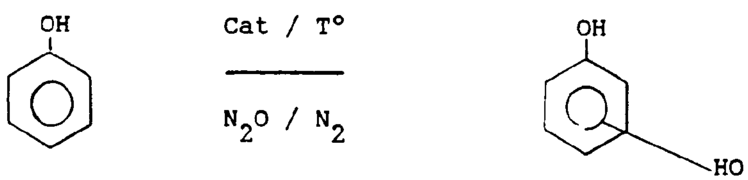


| | | |
|---|---|---|
| CONDITIONS EXPERIMENTALES | : | |
| Phase vapeur | : | continue |
| Catalyseur | : | . H Z S M-5 |
| | | . diam. pores : 550 pm |
| | | . Rapport $\frac{SiO_2}{Al_2O_3}$ = 120 |
| Température | : | . 400°C et 450°C |
| Temps de contact | : | . environ 1 seconde |
| Rapport molaire | : | . phénol / $N_2$ / $N_2O$ |
| | | 2 5 8 |

1,05 g de catalyseur HZSM5 ($SiO_2/Al_2O_3$ = 120) en poudre, dispersé dans 4 g de quartz en grains (< 0,8 mm) sont introduits dans un réacteur tubulaire en quartz (longueur = 16cm, diamètre intérieur = 1,8 cm).

On ajoute ensuite un lit de 10 cm de billes de verre permettant d'homogénéiser le mélange gazeux. Le réacteur ainsi chargé est conditionné 15 heures à 350°C sous azote dans un four tubulaire.

La réaction est effectuée en continu en introduisant 1,4 $cm^3/h$ de phénol, 1,44 l/h de protoxyde d'azote et 0,9 l/h d'azote.

Les résultats sont reportés dans le tableau I.

TABLEAU I.

| Ex | T° | TT PHENOL % | RT (TOTAL) dihydroxybenzène | REPARTITION ISOMERIQUE | | |
|---|---|---|---|---|---|---|
| | | | | ORTHO | META | PARA |
| 1 | 400°C | 5 | 87 | 30 | 33 | 37 |
| 2 | 450°C | 4,5 | 82 | 30 | 40 | 30 |

## Revendications

1. Procédé de préparation de dihydroxybenzènes caractérisé en ce qu'on met en présence du phénol et du protoxyde d'azote sur une zéolithe acidifiée.

2. Procédé selon revendication 1 caractérisé en ce que la zéolithe est choisie parmi les zéolithes HZSM-5, HY et H-Mordénite.

3. Procédé selon revendication 1 caractérisé en ce que la zéolithe est acidifiée par passage d'un acide inorganique

4. Procédé selon revendication 1 caractérisé en ce que le protoxyde d'azote est utilisé selon un rapport molaire calculé par rapport au phénol compris entre 1 et 10.

5. Procédé selon revendication 1 caractérisé en ce que la réaction est mise en oeuvre à une température comprise entre 300 et 500°C.

## Patentansprüche

1. Verfahren zur Herstellung von Dihydroxybenzolen, dadurch gekennzeichnet, daß man Phenol und Stickoxydul über einem angesäuerten Zeolith zusammenbringt.

2. Verfahren nach Anspruch 1, dadurch gekennzeichnet, daß der Zeolith unter den Zeolithen HZSM-5, HY und H-Mordenit ausgewählt wird.

3. Verfahren nach Anspruch 1, dadurch gekennzeichnet, daß der Zeolith durch Hindurchleiten einer anorganischen Säure angesäuert wird.

4. Verfahren nach Anspruch 1, dadurch gekennzeichnet, daß das Stickoxydul in einem Molverhältnis, berechnet auf Phenol, von 1 bis 10 eingesetzt wird.

5. Verfahren nach Anspruch, dadurch gekennzeichnet, daß die Reaktion bei einer Temperatur im Bereich von 300 bis 500°C durchgeführt wird.

## Claims

1. Process for the preparation of dihydroxybenzenes, characterised in that the phenol and nitrous oxide are brought into contact on an acidified zeolite.

2. Process according to Claim 1, characterised in that the zeolite is selected from the zeolites HZSM-5, HY and H-Mordenite.

3. Process according to Claim 1, characterised in that the zeolite is acidified by passing an inorganic acid through it.

4. Process according to Claim 1, characterised in that the nitrous oxide is used in accordance with a molar ratio calculated in relation to the phenol of between 1 and 10.

5. Process according to Claim 1, characterised in that the reaction is carried out at a temperature of between 300 and 500°.